# EUROPEAN PATENT SPECIFICATION

(11) **EP 2 594 282 B1**
(45) Date of publication and mention of the grant of the patent: **23.07.2014**
(21) Application number: 11189879.7
(22) Date of filing: 21.11.2011
(51) Int. Cl.: A61K 38/40, A61P 25/00

(54) **Lactoferrin and the white matter**
Lactoferrin und die weiße Substanz
Lactoferrine et substance blanche

(43) Date of publication of application: 22.05.2013
(73) Proprietor: Nestec S.A., 1800 Vevey (CH)
(72) Inventor: Wang, Bing, 300041 Tianjin (CN); Faure, Magali, 1072 FOREL (CH)
(74) Representative: Cogniat, Eric Jean Marie

(56) References cited:
- WO-A1-2010/130641
- WO-A1-2010/130646
- US-A1- 2008 003 330
- HUANG RONGQIN ET AL: "Neuroprotection in a 6-hydroxydopamine-lesioned Parkinson model using lactoferrin-modified nanoparticles", JOURNAL OF GENE MEDICINE, JOHN WILEY & SONS, INC, US, vol. 11, no. 9, 1 September 2009 (2009-09-01), pages 754-763, XP002549625, ISSN: 1099-498X, DOI: 10.1002/JGM.1361 [retrieved on 2009-06-24]
- GUANGLIN ZHANG ET AL: "Gypenoside attenuates white matter lesions induced by chronic cerebral hypoperfusion in rats", PHARMACOLOGY BIOCHEMISTRY AND BEHAVIOR, ELSEVIER, US, vol. 99, no. 1, 25 March 2011 (2011-03-25) , pages 42-51, XP028214177, ISSN: 0091-3057, DOI: 10.1016/J.PBB.2011.03.019 [retrieved on 2011-04-01]

## Description

The present invention generally relates to brain white matter. In particular, the present invention relates to a composition that can be used for promoting the development and/or avoids loss of white matter. One embodiment of the present invention relates to a composition comprising lactoferrin for use in the promotion of the development of the white matter, in the treatment or prevention of a delayed development of the white matter, and or in the treatment of prevention of a loss of white matter.

Nutrients are the prerequisite for growth and development of an organism. "Building blocks" of newly laid down tissues can derive from the diet as preformed units or can be obtained via endogenous biosynthesis or bioconversion. This process occurs at maximum rates when tissue growth is fastest, namely during the intrauterine period.

After tissue formation all tissues undergo a variable degree of remodeling (or turnover) but remodeling is a rather slow process especially for the central nervous system. Sorting out the proportions of the two main sources of non-indispensable or semi-indispensable building blocks for tissue growth is not a simple task since (a) tissue growth is a rather slow in general and (b) building blocks from a dietary source are hard to differentiate from those of endogenous biosynthesis.

This issue is however of paramount importance in human nutrition in general and in infant nutrition in particular. During phases of rapid growth the availability of non-indispensable or semi-indispensable nutrients may influence the endogenous biosynthesis and may limit the growth potential of the foetus or of the newborn infant.

Pregnant and lactating women and young children less than three years are most vulnerable to malnutrition. Scientific evidence has shown that there are adverse neurological consequences of early -life chronic malnutrition, such as poor spatial navigation, memory formation and memory consolidation.

Individuals better nourished in the first three years of life have dramatically better lives. Nutrients passing the placental barrier during intrauterine development and nutrients, which are present in human milk, may represent the most appropriate candidates to improve early brain development, particularly following adverse prenatal exposures.

Proper brain development is a key concern of all parents.

The human brain consists of grey matter, white matter and substantia nigra. The grey matter is made up of neuronal cell bodies and includes regions of the brain involved in memory, seeing, hearing, speech, but also emotions and muscle control. The substantia nigra plays a role in learning, aiming for rewards and addiction.

According to Reiss et al., Brain, 1995, Volume 119, Issue 5, pp. 1763-1774, IQ is positively correlated with total cerebral volume in children, in particular, with the volume of cortical grey matter in the prefrontal region of the brain. Subcortical grey matter was also found to contribute to the variance in IQ, although to a lesser extent than cortical grey volume. Consequently, there is an effort in the art to promote the development of grey matter.

White matter is the tissue through which messages pass between different areas of gray matter within the nervous system. Myelin surrounds long nerve fibers and provides electrical insulation allowing a fast and efficient message transfer. Myelination speeds up neuronal transmission by the elaboration of a concentric phospholipid layer of insulation around axons by oligodendrocytes. Maturation of brain white matter pathways is an important factor in cognitive, behavioral, emotional and motor development in children.

White matter integrity consisting of greater functional connectivity that may contribute to the establishment of widely distributed brain functions that are believed to underlie complex voluntary control of behavior ( Luna B. in: Aboitiz F, et al., editors. From attention to goal-directed behavior: neurodynamical, methodological and clinical trends. Heidelberg (Germany): Springer-Verlag; 2009. p. 249-274). A fast and efficient message transfer is also very important for a proper brain performance. Hence there is a strong need in the art to also support the development of white matter.

The present inventors have addressed this need.

Consequently, it was the objective of the present invention to provide the art with a composition that specifically allows it to promote the development of white matter and/or that allows it to catch up after a phase of slower development of the white matter. It would further be desirable if this composition could also be used to support the development of unborn babies through maternal nutrition.

The inventors were surprised to see that they could achieve these objectives by the subject matter of the independent claims. The dependant claims further develop the idea of the present invention.

Using the powerful high field MRI technique, the inventors were able to demonstrate that maternal lactoferrin supplementation resulted in a significant increase in brain white matter volume.

This was for example shown for the white matter volume of the corpus callosum content.

Effects were seen, for example, for intrauterine growth restriction (IUGR) mammals. Intrauterine Growth Restriction (IUGR), previously known as intrauterine growth retardation, is more frequently seen at the end of a pregnancy and may have negative consequences for the development of the infant. The likelihood of IUGR can be increased by several factors, such as alcohol abuse, clotting disorders, drug addiction, high blood pressure or heart disease, kidney diseases, poor nutrition, smoking, infection and maternal illnesses.

Fetal risk factors that may contribute to IUGR include genetic issues or a multiple pregnancy, for example.

Risk factors in the placenta and umbilical cord abnormalities that may contribute to IUGR include: Placenta previa, accreta, or abruption, along with other placental abnormalities. A further risk factor is the location of the umbilical cord around neck or a part of body, which decreases umbilical cord blood flow.

Lactoferrin, a serum sialylated glycoprotein is secreted by humans from the embryo to the adult stage, and is especially abundant in colostrum and milk. This sialylated glycoprotein has various physiological effects including, regulation of iron homeostasis, cell growth and differentiation, prebiotic and anti-inflammatory actions and stimulation of the immune response.

The inventors have shown that in animals with a negatively altered brain structure and development maternal lactoferrin supplementation throughout gestation and lactation could revise and/or restore the alteration in brain structure and/or development.

Scientific evidence is provided that lactoferrin supplementation increases the brain white matter volume, e.g., of corpus callosum content in intrauterine growth restriction (IUGR) mammals and supports catching up to normal development during early life.

Consequently, the present invention relates in part to a composition comprising lactoferrin for use in the promotion of the development of the white matter and/or in the treatment or prevention of a delayed development of the white matter.

The present invention also relates to the use of lactoferrin in the preparation of a composition for the promotion of the development of the white matter and/or for the treatment or prevention of a delayed development of the white matter.

Lactoferrin (LF), also known as lactotransferrin (LTF), is a globular multifunctional protein that is known to exhibit an antimicrobial activity and is a part of the innate defense, mainly at mucoses.

Lactoferrin may be found for example in milk and whey and in many mucosal secretions such as tears and saliva. As such, Lactoferrin may be purified, e.g., from milk or may be produced recombinantly.

The present invention relates to lactoferrin obtainable from any source.

Lactoferrin from milk or whey, for example, has the advantage that it is a natural ingredient obtained from a food-grade composition and can consequently be used as enriched fraction of the food composition without further purification.

The composition of the present invention may consequently comprise lactoferrin provided as milk or whey fraction enriched in lactoferrin.

Enriched means that lactoferrin was either added to the fraction, so that the resulting lactoferrin content of the fraction is higher than the lactoferrin content of the fraction without lactoferrin addition, or that milk or whey was treated in a way to concentrate the natural lactoferrin content in a fraction.

Recombinantly obtained lactoferrin has the advantage that it can be produced easily in high concentrations.

Human colostrum has a relatively high concentration of lactoferrin, followed by human milk, then cow milk.

In therapeutic applications, compositions are administered in an amount sufficient to at least partially cure or arrest the symptoms of the disorder and/or its complications. An amount adequate to accomplish this is defined as "a therapeutically effective dose". Amounts effective for this purpose will depend on a number of factors known to those of skill in the art such as the severity of the disorder and the weight and general state of the patient. In prophylactic applications, compositions according to the invention are administered to a patient susceptible to or otherwise at risk of a particular disorder in an amount that is sufficient to at least partially reduce the risk of developing a disorder. Such an amount is defined to be "a prophylactic effective dose". Again, the precise amounts depend on a number of patient specific factors such as the patient's state of health and weight.

Lactoferrin may be administered in the framework of the present invention in a therapeutically effective dose and/or in a prophylactic effective dose.

Typical compositions comprising lactoferrin in accordance with the present invention may comprise lactoferrin in an amount of at least 1,6 g/L.

For example, the composition of the present invention may contain lactoferrin in a concentration of at least 0.75% (w/w), preferably at least 1% (w/w).

In one embodiment, the composition is to be administered in an amount corresponding to an ingestion of at least 0.01g lactoferrin, at least 0.25g lactoferrin, preferably at least 0.5 g lactoferrin more preferably at least 1 g lactoferrin per day per kg body weight per day.

For example, the composition may be consumed in an amount corresponding to at least 1g lactoferrin/kg body weight/day intake for pregnant and/or lactating mothers.

The composition may also be consumed in an amount corresponding to at least 200mg lactoferrin/kg body weight/day intake for the children.

Lactoferrin may be present in the composition in a concentration of at least 0,01 g per 100kcal, preferably of at least 0,1 g per 100kcal. For example, lactoferrin may be present in the composition in the range of about 0,01g -100g, preferably 0,1g - 50 g, even more preferred 2 g - 25 g per 100 kcal of the composition.

Lactoferrin may also be used in combination with other compounds, such as sialic acid and/or iron, for example.

The composition of the present invention may for example comprise at least about 0.001 weight-% sialic acid. In further embodiments of the present invention, the composition may comprise at least about 0.005 weight-%, or at least about 0.01 weight-% of sialic acid.

Alternatively or additionally the lactoferrin containing composition may contain iron in an amount in the range of about 1mg/100g (w/w) to 50mg/100g (w/w) of the composition, for example 10 mg/100g (w/w) to 30 mg/100g (w/w) of the composition.

A good source for lactoferrin is whey protein. The composition of the present invention may consequently comprise a protein source containing at least 50% by weight whey protein.

White matter is a component of the central nervous system and contains large amounts of myelinated axons. White matter forms the bulk of the deep parts of the brain and the superficial parts of the spinal cord.

Consequently, the white matter that is treated with the composition of the present invention may be the white matter of the brain, for example the white matter of the corpus callosum. The corpus callosum is the largest white matter structure in the brain connecting the left and right cerebral hemispheres and facilitating interhemispheric communication. The corpus callosum is a wide, flat bundle of neural fibers beneath the cortex in the eutherian brain at the longitudinal fissure and is also known as colossal commissure.

With the composition of the present invention, it is for example possible to increase the volume of white matter.

Work of Marner et. al., 2003, J. Comp. Neurol. 462(2):144-52 has shown that men have slightly more white matter than women both in volume and in length of myelinated axons. At the age of 20, the total length of myelinated fibers in males was found to be 176,000 km while that of a female was found to be 149,000 km. The authors have observed a decline in total length with age of about 10% each decade such that a man at 80 years of age had 97,200 km and a female 82,000 km.

Consequently, the composition of the present invention can be used to treat, prevent or ameliorate an age related decline of white matter.

As age related decline of white matter starts at about an age of 20 years, the composition of the present invention may be to be administered to subjects of at least 20 years, at least 30 years, at least 40 years, at least 50 years, at least 60 years, at least 70 years or at least 80 years.

The composition of the present invention may be any composition suitable for oral, enteral or parenteral administration.

For example, the composition may be selected from the group consisting of food products, animal food products, pharmaceutical compositions, nutritional formulations, nutraceuticals, drinks, food additives, infant feeding formulas, or maternal food compositions.

The composition of the present invention may be to be administered to anyone in need thereof, for example to infants, children and teenagers to support white matter development and to adults or elderly to treat or prevent age related loss of white matter.

For example, the composition of the present invention may be to be administered to mothers during pregnancy, mothers during lactation, premature or term born babies, infants, toddlers, children and/or teenagers.

In particular, lactoferrin and/or the composition of the present invention may be administered for example to
- premature or term-born infants having experienced an intrauterine growth retardation that may occur following any adverse events during the gestation (smoking of the mother, medication of the mother, low placenta quality, abnormal placenta positioning, malnutrition of the mother and the fetus, etc)
- premature infants without any intrauterine growth retardation
- very low / low birth weight infants
- IUGR infants
- neonates and children showing brain growth retardation following for example hypoxemia-ischemia at birth or any other adverse event
- neonates and children showing cognitive dysfunctions, retardation.

Lactoferrin or the composition of the present invention can therefore be administered to the child and/or to the mother during the gestation and/or lactation period.

For the purpose of the present invention, the term "child" includes infants and comprises subjects in the age range from 0-14 years.

"Infants" are children under the age of 12 months.

A human infant less than a month old is a newborn or a neonate. The term "newborn" includes premature infants, postmature infants, low birth weight infant and full term newborns. Upon reaching the age of one or beginning to walk, infants are also referred to as "toddlers" (generally 12-36 months).

In one embodiment of the present invention the composition is to be administered to mothers with IUGR infants or to IUGR infants.

The composition of the present invention can be in particular effective in IUGR mammals. Intrauterine Growth Restriction (IUGR) is a term used to describe a condition in which the fetus or infant is smaller than expected for the number of weeks of pregnancy. A fetus or infant with IUGR often has a weight that is reduced by at least 10% compared to normal fetusses or infants of the same gestational age. For example a human fetus with IUGR may be born at term (after 37 weeks of pregnancy) or prematurely (before 37 weeks).

The composition described in the present invention may be for use in the treatment, prevention or amelioration of white matter disorders.

In many neurological disorders in childhood, the white matter of the brain is predominantly involved. These are referred to as "white matter disorders". They are commonly diagnosed based on MRI findings. White matter abnormalities on MRI can have many different reasons on the tissue level. For example, there may be a lack of myelin and/or myelin may be degraded. Innumerable vacuoles may have formed within the myelin sheath or scar tissue may have formed within the white matter. There may be increased amounts of water between the myelinated fibers, etc, and/or nerve fibers may be lost.

Consequently, "white matter disorders" comprises many different disorders which may have consequences for brain function of varying severity.

Might matter disorders may be hereditary or acquired.

The causes of the acquired forms of white matter disorders can be subdivided according to type of cause into infectious-inflammatory, noninfectious-inflammatory, toxic-metabolic, traumatic and hypoxic-ischemic.

The following disorders may be acquired white matter disorders, for example
Non-infectious -inflammatory disorders, such as
   Multiple sclerosis and its variants
   Acute disseminated encephalopmyelitis
Infectious-inflammatory disorders, such as
   Congenital CMV
   Subacute sclerosing panencephalitis
   Subacute HIV encephalitis
   Subacute CMV encephalitis
   Progessive multifocal leukoencephalitis
Toxic-metabolic disorders, such as
   Central pontine and extrapontine myelinolysis
   Vitamin B12 deficiency
   Folate deficiency
   Marchiafava-Bignami disease
   Malnutrition
Toxic encephalopathies (endogenous - exogenous) Hypoxic-ischemic disorders, such as
   Periventricular leukomalacia
   Polycystic leukoencephalopathy
   Delayed posthypoxic-ischemic demyelination
   Subcortical arteriosclerotic encephalopathy (Binswanger)
   Aging related
   Vasculitis
   Radiation
Traumatic disorders, such as
   Shearing
   Edema

Because of the multitude of genes and proteins involved in the different disorders, the classification of hereditary white matter disorders is complex and is often made based on the biochemical pathway that involved.

The following disorders may be hereditary white matter disorders, for example:
Lysosomal disorders
   Metachromatic leukodystrophy
   Krabbe's disease (globoid cell leukodystrophy)
   GM1 gangliosidosis (infantile variant)
   GM2 gangliosidosis (infantile variant)
   Infantile neuronal ceroid lipufuscinosis
   Fabry's disease
   Sialic acid storage disorder, Salla disease
   Fucosidosis
   Mucoploysaccharidoses
Peroxisomal disorders
   Zellweger syndrome
   Neonatal adrenoleukodystrophy
   Infantile Refsum disease
   Zellweger-like syndrome
   Pseudo-Zellweger syndrome
   Pseudo-neonatal adrenoleukodystrophy
   Bifunctional protein deficiency
   X-linked adrenoleukodystrophy and adrenomyeloneuropathy
   Refsum disease
Mitochondrial disorders
   Respiratory chain defects
   Cerebrotendinous xanthomatosis
DNA repair disorders
   Cockayne's disease
   PIBD or Tay syndrome
Defects in myelin proteins
   Pelizaeus-Merzbacher disease
   18q- syndrome
   Amino acidopathies and organic acidopathies
   Phenylketonuria
   Glutaric aciduria type I
   Propionic aciduria
   Nonketotic hyperglycinemia
   Maple syrup urine disease
   Canavan's disease
   L-2-hydroxyglutaric aciduria
   Hyperhomocysteinemias
   Urea cycle defects
   3-hydroxy-3-methylglutaryl Coenzyme A lyase deficiency
   Serine synthesis defect
Further disorders
   Galactosemia
   Sjögren-Larsson syndrome
   Lowe syndrome
   Wilson disease
   Alexander disease
   Myotonic dystrophy
   Congenital muscular dystrophies
   Megalencephalic leukoencephalopathy with subcortical cysts
   Vanishing white matter / CACH
   Hypomyelination with atrophy of basal ganglia and cerebellum
   Aicardi-Goutières syndrome

Further disorders that may be white matter disorders include vanishing white matter disease, leukoencephalopathy with brain stem and spinal cord involvement and elevated lactate, megalencephalic leukoencephalopathy with subcortical cysts, hypomyelination, hypogonadotropic hypogonadism and hypodontia, hypomyelination with atrophy of the basal ganglia and cerebellum, and defects in polyol metabolism.

Those skilled in the art will understand that they can freely combine all features of the present invention described herein, without departing from the scope of the invention as disclosed. In particular, features described for the uses of the present invention may be applied to the composition of the present invention and vice versa.

Further advantages and features of the present invention are apparent from the following Examples and Figures.

Figure 1 shows the acquisition of DT images of slices positioned all the along the corpus callosum (CC) for excised brains from 21 day-old pups.

Figure 2 shows the quantitative results of the assessment of the microstructure of the brain tissue using advanced high field 9.4T MR Imaging called Diffusion Tensor Imaging (DTI) at postnatal day (PND) 21.

### Examples:

To demonstrate the effect of lactoferrin on the white matter of the brain, the inventors have selected a well-accepted and challenging model of maternal supplementation. It was found that, e.g., maternal lactoferrin supplementation had significant benefits on neuronprotection and neurodevelopment through increase of the brain white matter volume of corpus callosum content, for example in intrauterine growth restriction (IUGR) mammals.

A model of intrauterine growth retardation (IUGR) was obtained by the treatment of dams with dexamethasone (100µg/kg/day) during the third week of gestation. Lactoferrin was orally administrated to dam *ad libitum* at dose level of 1g/kg/day from the beginning of gestation to the end of the lactation (6 weeks).

The following 3 groups of animals were tested:
Group 1 : Normal pups; no lactoferrin intervention in control dams (sham = osmotic pump with saline buffer).
Group 2: IUGR pups; no lactoferrin intervention in DEX treated dams.
Group 3: IUGR pups; bLf supplementation (1g/Kg/day) of DEX treated dams from the beginning of gestation to the end of the lactation.

The microstructure of the brain tissue was assessed *ex vivo* using advanced high field 9.4T MR Imaging called Diffusion Tensor Imaging (DTI) at postnatal day (PND) 21.

Two groups of IUGR pups and one normal group underwent *ex vivo* assessment of the microstructure of the brain at postnatal day 21.

At PND 21 animals were perfused intracardially with 0.9% NaCl, then 4% paraformaldehyde. Brains were removed, weighed and postfixed in 4% paraformaldehyde overnight, then stored at +4°C in 1X Phosphate Buffered Saline. *Ex-vivo* DTI experiments were performed with a transceive 25-mm birdcage RF coil. First and second order shims were manually adjusted, with water bandwidth ranged between 20 to 40 Hz. A Spin Echo sequence with addition of the Stejskal-Tanner diffusion gradients was used. Diffusion gradients were applied along twelve spatial directions: (x, y, 0), (x, -y, 0), (x, 0, z) , (x, 0, -z), (0, y, z), (0, y, -z) as well as the six opposite directions to cancel b-value cross terms. Intensity, duration and diffusion time were set to 22 G/cm, 3 ms and 20 ms respectively, given a b-value of 1184 s·mm². A field of view of 17 × 17 mm² was sampled on a 128 × 64 cartesian grid zero filled to 256 × 256, given an in-plane pixel size of 66µm. 15 slices of 0.5 mm thickness were acquired in the axial plane. Scans were averaged 10 times with TE/TR = 35/2000 ms. Data analysis: Using homemade Matlab (Mathworks, Natick, MA) software, the diffusivity values (Dₘᵢₙ, D_{med} and Dₘₐₓ) were derived from the tensor. The orthogonal diffusivity (Dₒᵣₜₕ= (Dₘᵢₙ, D_{med})/2), the parallel diffusivity (D// = Dₘₐₓ), the mean diffusivity (DAV = (Dₘᵢₙ + D_{med} + Dₘₐₓ)/3) and the fractional anisotropy (FA = [(3/2) × ((Dₘᵢₙ - MD) ² + (D_{med} - MD)² + (Dmax - MD)²) / (Dₘᵢₙ² + D_{med}² + Dₘₐₓ²)]1/2) were computed.

The program allows manual delineation of region of interest (ROI) on the FA maps. Two different regions of the brain were analyzed: the corpus callosum (CC) and the Cortex (CX) at 5 different image-planes of the brain.

### Results

### MRI

New MRI hardware (antenna and holder) have been developed for this project. In combination with an optimized sequence for diffusion tensor imaging, the gain of resolution compared to the previous protocol is about 3.6-fold (resolution increase from 2.10-2 mm³ to 5.5.10-3mm³).

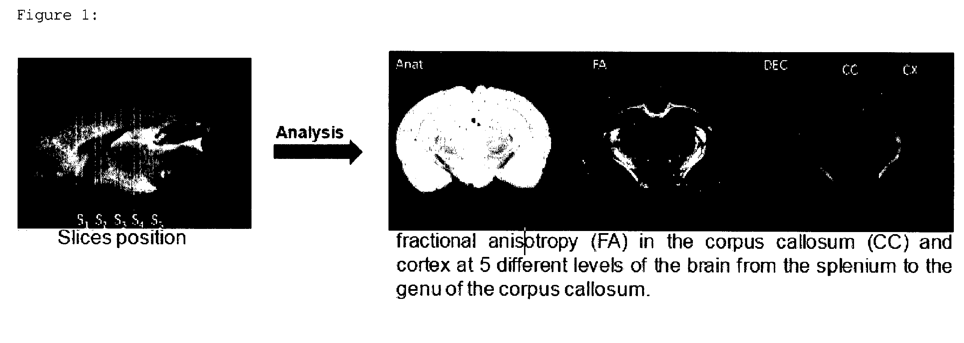

Acquisition of DT images of slices positioned all the along the corpus callosum (CC) has been done for excised brains from 21 day-old pups. Using fractional anisotropy (FA) parameter, the integrity of cortical structure and fibre tracts (CC) were compared between the different groups at PND 21 (n = 6 for all groups) (Fig 1).

Cortex: No differences of FA value were observed.

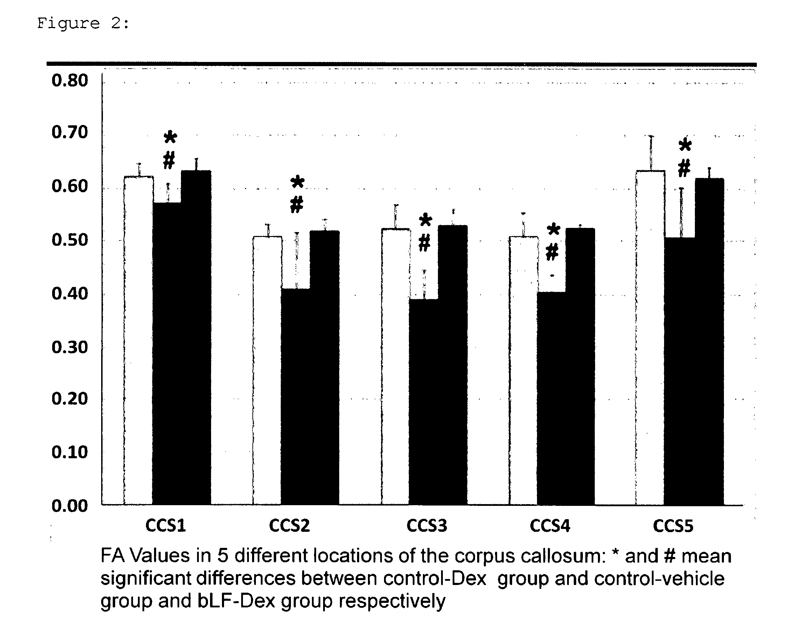

A significant decrease of FA value of the control-Dex group compared with the control-vehicule group was measured in the corpus callosum (Figure 2). It is due to a significant increase of Dₒᵣₜₕ that could relate to a myelination defect. Pups exposed to dam lactoferrin supplementation have shown restored FA values in their corpus callosum (Fig 2). The corpus callosum is the largest white matter structure in the brain, consisting of 200-250 million contralateral axonal projections that are responsible for connecting the left and right cerebral hemispheres and facilitating interhemispheric communication.

In conclusion, prenatal Dex exposure induced alteration in brain structure and development. Maternal lactoferrin supplementation throughout gestation and lactation could revise (restore) the prenatal Dex exposure induced alteration in white matter brain structure and promote the development of white matter brain structure. These findings are strong evidence that lactoferrin supplementation has benefits on neuroprotection and brain maturation processes, e.g., in children.

## Claims

1. Composition comprising lactoferrin for use in the promotion of the development of the white matter, in the treatment or prevention of a delayed development of the white matter, and or in the treatment of prevention of a loss of white matter.

2. Composition for use in accordance with claim 1, wherein the composition comprises lactoferrin in an amount of at least 0.1g/100 kcal of the composition.

3. Composition for use in accordance with one of the preceding claims, wherein lactoferrin is present in a concentration in the range of about O,Olg -100g, preferably O,lg - 50 g, even more preferred 2 g - 25 g per 100 kcal.

4. Composition for use in accordance with one of the preceding claims, wherein the composition is to be administered in an amount corresponding to an ingestion of at least 0.01g lactoferrin per kg body weight per day.

5. Composition for use in accordance with one of the preceding claims, wherein the lactoferrin is provided as a milk or whey fraction enriched in lactoferrin.

6. Composition for use in accordance with one of the preceding claims, wherein the composition comprises a protein source containing at least 50% by weight whey protein.

7. Composition for use in accordance with one of the preceding claims, wherein the white matter is the white matter of the brain.

8. Composition for use in accordance with one of the preceding claims, wherein the white matter is the white matter of the corpus callosum.

9. Composition for use in accordance with one of the preceding claims, wherein the volume of the white matter is increased.

10. Composition for use in accordance with one of the preceding claims, wherein the composition is selected from the group consisting of food products, animal food products, pharmaceutical compositions, nutritional formulations, nutraceuticals, drinks, food additives, infant feeding formulas, or maternal food compositions.

11. Composition for use in accordance with one of the preceding claims, wherein the composition is to be administered to mothers during pregnancy, mothers during lactation, premature or term born babies, infants, toddlers, children and/or teenagers.

12. Composition for use in accordance with one of the preceding claims, wherein the composition is to be administered to mothers with IUGR infants or to IUGR infants.

13. Composition for use in accordance with one of the preceding claims for the treatment or prevention of white matter disorders.

14. Composition for use in accordance with claim 13, wherein the white matter disorder is hereditary or acquired.

## Patentansprüche

1. Zusammensetzung mit Lactoferrin zur Verwendung in der Förderung der Entwicklung der weißen Substanz, in der Behandlung oder Vorbeugung einer verzögerten Entwicklung der weißen Substanz, und/oder in der Behandlung oder Vorbeugung eines Verlustes der weißen Substanz.

2. Zusammensetzung zur Verwendung nach Anspruch 1, wobei die Zusammensetzung Lactoferrin in einer Menge von mindestens 0,1 g/100 kcal der Zusammensetzung aufweist.

3. Zusammensetzung zur Verwendung nach einem der vorhergehenden Ansprüche, wobei Lactoferrin in einer Konzentration im Bereich von ca. 0,01 g - 100 g, vorzugsweise 0,1 g - 50 g, weiter bevorzugt 2 g - 25 g, pro 100 kcal vorhanden ist.

4. Zusammensetzung zur Verwendung nach einem der vorhergehenden Ansprüche, wobei die Zusammensetzung in einer Menge zu verabreichen ist, die einer Aufnahme von mindestens 0,01 g Lactoferrin pro kg Körpergewicht pro Tag entspricht.

5. Zusammensetzung zur Verwendung nach einem der vorhergehenden Ansprüche, wobei das Lactoferrin als Milch oder Molkeanteil, angereichert mit Lactoferrin, bereitgestellt ist.

6. Zusammensetzung zur Verwendung nach einem der vorhergehenden Ansprüche, wobei die Zusammensetzung eine Proteinquelle aufweist, die mindestens 50 Gew.-% Molkenprotein enthält.

7. Zusammensetzung zur Verwendung nach einem der vorhergehenden Ansprüche, wobei die weiße Substanz die weiße Substanz des Gehirns ist.

8. Zusammensetzung zur Verwendung nach einem der vorhergehenden Ansprüche, wobei die weiße Substanz die weiße Substanz des Balkens (*corpus callosum*) ist.

9. Zusammensetzung zur Verwendung nach einem der vorhergehenden Ansprüche, wobei das Volumen der weißen Substanz erhöht ist.

10. Zusammensetzung zur Verwendung nach einem der vorhergehenden Ansprüche, wobei die Zusammensetzung aus der Gruppe ausgewählt ist, die folgendes aufweist:
Lebensmittelprodukte, Tiernahrung, pharmazeutische Zusammensetzungen, Nahrungsrezepturen, Nutrazeutika, Getränke, Nahrungsmittelzusätze, Säuglingsnahrung oder Nahrungsmittelzusammensetzungen für Mütter.

11. Zusammensetzung zur Verwendung nach einem der vorhergehenden Ansprüche, wobei die Zusammensetzung an Mütter während der Schwangerschaft, Mütter während der Stillzeit, Frühgeborene oder Neugeborene, Säuglinge, Kleinkinder, Kinder und/oder Teenager zu verabreichen ist.

12. Zusammensetzung zur Verwendung nach einem der vorhergehenden Ansprüche, wobei die Zusammensetzung an Mütter mit IUGR-Säuglingen (intrauterine Wachstumsrestriktion) oder an IUGR-Säuglinge zu verabreichen ist.

13. Zusammensetzung zur Verwendung nach einem der vorhergehenden Ansprüche zur Behandlung oder Vorbeugung von Störungen der weißen Substanz.

14. Zusammensetzung zur Verwendung nach Anspruch 13, wobei die Störung der weißen Substanz erblich oder erworben ist.

## Revendications

1. Composition comprenant de la lactoferrine pour une utilisation pour favoriser le développement de la substance blanche, dans le traitement ou la prévention d'un développement tardif de la substance blanche, et ou dans le traitement ou la prévention d'une perte de substance blanche.

2. Composition pour une utilisation selon la revendication 1, dans laquelle la composition comprend de la lactoferrine dans une quantité d'au moins 0,1 g/100 kcal de la composition.

3. Composition pour une utilisation selon l'une des revendications précédentes, dans laquelle la lactoferrine est présente dans une concentration dans la gamme d'environ 0,01g - 100g, de préférence 0,1g - 50 g, encore plus préférablement 2 g - 25 g par 100 kcal.

4. Composition pour une utilisation selon l'une des revendications précédentes, dans laquelle la composition est destinée à être administrée dans une quantité correspondant à une ingestion d'au moins 0,01g de lactoferrine par kg de poids corporel par jour.

5. Composition pour une utilisation selon l'une des revendications précédentes, dans laquelle la lactoferrine est fournie en tant que fraction de lait ou de lactosérum enrichie en lactoferrine.

6. Composition pour une utilisation selon l'une des revendications précédentes, dans laquelle la composition comprend une source de protéine contenant au moins 50% en poids de protéine de lactosérum.

7. Composition pour une utilisation selon l'une des revendications précédentes, dans laquelle la substance blanche est la substance blanche du cerveau.

8. Composition pour une utilisation selon l'une des revendications précédentes, dans laquelle la substance blanche est la substance blanche du corps calleux.

9. Composition pour une utilisation selon l'une des revendications précédentes, dans laquelle le volume de la substance blanche est augmenté.

10. Composition pour une utilisation selon l'une des revendications précédentes, dans laquelle la composition est choisie parmi le groupe constitué de produits alimentaires, produits alimentaires pour animaux, compositions pharmaceutiques, formulations nutritionnelles, nutraceutiques, boissons, additifs alimentaires, formules d'alimentation de nourrissons, compositions alimentaires maternelles.

11. Composition pour une utilisation selon l'une des revendications précédentes, dans laquelle la composition est destinée à être administrée à des mères au cours de la grossesse, des mères au cours de la lactation, des bébés prématurés ou nés à terme, des nourrissons, des enfants en bas âge, des enfants et/ou des adolescents.

12. Composition pour une utilisation selon l'une des revendications précédentes, dans laquelle la composition est destinée à être administrée à des mères avec des nourrissons à RCIU (retard de croissance intra utérin) ou à des nourrissons à RCIU.

13. Composition pour une utilisation selon l'une des revendications précédentes pour le traitement ou la prévention de troubles de la substance blanche.

14. Composition pour une utilisation selon la revendication 13, dans laquelle le trouble de la substance blanche est héréditaire ou acquis.
